# EUROPÄISCHE PATENTSCHRIFT

(11) **EP 0 767 670 B1**
(45) Veröffentlichungstag und Bekanntmachung des Hinweises auf die Patenterteilung: **21.08.2002**
(21) Anmeldenummer: 95925777.5
(22) Anmeldetag: 28.06.1995
(51) Int. Cl.: A61K 35/54, A61P 3/14

(54) **PUTAMEN OVI**
PUTAMEN OVI
PREPARATION A BASE DE COQUILLES D'OEUFS DITE "PUTAMEN OVI"

(30) Priorität: 28.06.1994 DE 4422613
(43) Veröffentlichungstag der Anmeldung: 16.04.1997
(73) Patentinhaber: aar Pharma GmbH & Co. KG, 42853 Remscheid (DE)
(72) Erfinder: RUEPP, Michel O., 42853 Remscheid (DE)
(74) Vertreter: Jönsson, Hans-Peter, Dr.Dipl.-Chem.
(86) Internationale Anmeldenummer: EP9502513
(87) Internationale Veröffentlichungsnummer: WO96000578

(56) Entgegenhaltungen:
- EP-A- 0 347 899
- US-A- 3 558 771
- DATABASE WPI Section Ch, Week 8437 Derwent Publications Ltd., London, GB; Class B04, AN 84-229077 & JP,A,59 137 415 ( ONODA M) , 7.August 1984
- DATABASE WPI Section Ch, Week 9513 Derwent Publications Ltd., London, GB; Class B06, AN 95-091262 & CN,A,1 079 149 ( LI H) , 8.Dezember 1993

## Beschreibung

Gegenstand der Erfindung ist ein Verfahren zur Herstellung von Putamen ovi, Putamen ovi einer definierten Korngrößenverteilung sowie die Verwendung von Putamen ovi zur Behandlung von verschiedenen Krankheiten.

Putamen ovi im Sinne der vorliegenden Erfindung umfaßt hygienisch aufbereitete Eischale, insbesondere von gallus domesticus.

Nach Römpp Chemielexikon, 9. Auflage, 1990, Seite 1079, Stichwort "Eier" ist die Eischale bei Hühnereiern 0,2 bis 0,4 mm dick und je nach Rasse weiß oder braun gefärbt. Sie setzt sich aus einem Proteingerüst (Protein-Mucopolysaccharid-Komplex) zusammen, in das Calciumcarbonat sowie geringere Menge an _{Ca}und Mg-Salze eingelagert sind. Die Schale enthält Poren (7000-17000 pro Ei), die von Proteinfasern erfüllt sind. Die Eier anderer Vogelarten wie Gans, Ente, Taube oder Wachtel, treten im Vergleich zu Hühnereiern stark zurück und werden stets nach der Herkunft bezeichnet. Die Schale enthält größenordnungsmäßig 98,4 % Trockenmasse, bestehend aus 3,3 % Proteinen und 95,1 % Mineralstoffen.

Aus SU 1 754 104 ist die Anwendung von Eischalen als Zahnputzmittel bekannt. Die Anwendung dieses Präparates erfolgt als Zahnputzmittel. Es enthält einen angeblichen Kariesinhibierenden Film mit reduzierten abrasiven Eigenschaften. Dieses Zahnputzmittel enthält lediglich einen sehr geringen nicht wirkstoffbestimmenden Anteil an Eischalen - als Adjuvanz. Das Eischalenpulver liegt hier nicht als Monosubstanz vor, sondern eingebettet in Natriumbicarbonat 35-45 % (m/m) - und wird nicht vom Organismus aufgenommen.

In CH 193 065 A wird ein flüssiges Tonikum beschrieben, das eigelbreich - damit besonders cholesterinreich -, stark zuckerund alkoholhaltig ist, jedoch wenig Eischalenbestandteile enthält. Dies ist aufgrund des Herstellungsverfahrens bedingt. Die fertige Emulsion enthält 2 - 3 % Eischalenbestandteile als Zitrate - jedoch nur diejenigen, die gelöst oder emulgiert werden. In diesem Präparat sind nur bestimmte Fraktionen der Eischale enthalten. Aufgrund des hohen Anteils an Cholesterin, Zucker und Alkohol ist dieses Tonikum hinsichtlich des deutlichen Belastungspotentials physiologischer Regelkreise therapeutisch nicht akzeptabel.

Gemäß der FR-A-0 649 055 werden die Eischalen bei 50 °C mit Ethylenoxid 20 % bei einem Druck von 5 Atmosphären sterilisiert. Dieses Verfahren ermöglicht eine Keimreduzierung - jedoch keine notwendige vollständige Sterilisation.

Das in GB 2 218 906 beschriebene Präparat wird zu Behandlung von Hautläsionen eingesetzt. Fein gemahlene Eischalen werden in eine bevorzugt flüssige orale oder topisch anzuwendende Rezeptur verarbeitet, die besonders auch Essenzen, Paraffin sowie verschiedene Wachse und Paraffinöle enthält. Die Anwendung dieses Präparates bei Ekzemen und allergisch-bedingten Hauterscheinungen ist unter oraler und topischer Anwendung nicht akzeptabel, da Eischalen aufgrund ihrer Proteinbasis selbst ein hohes allergenes Potential besitzen und typische Hautirritationen auslösen können, sowie bestehende Krankheitsbilder bestimmter Hautläsionen allergischer Natur verstärken können. Das Erhitzen der Eischalen zur Sterilisierung mit Hilfe der Mikrowelle über einen Zeitraum von 6 Minuten ist unzureichend um pathogene Erreger zu eliminieren.

In der EP 0 347 899 A2 wird ein Sterilisationsverfahren für Eischalen beschrieben. Das angegebene Sterilisierungsverfahren ist ungeeignet, eine mögliche Anwesenheit von pathogenen Bakterien, Sporen, Pilzen und Protozoen zu eliminieren. Die Sterilisation des Eischalenpulvers mit trockener Luft bei 120 °C etwa 1 Stunde lang ist nicht geeignet, um eine sichere Reduzierung pathogener Keime durchzuführen sowie einer Wirkstoffminderung aufgrund zu hoher Temperatur entgegenzuwirken. Eine Temperaturerhöhung >80°C- insbesondere im Bereich ≥150°C-über 1 Stunde lang, zerstört die biologisch vorhandenen membranpassage-fähigen Carrier für den wirksamen Mineralstofftransport in Kompakta und Spongiosa. Nach dieser thermischen Belastung zeigt das Eischalenpulver die biologischen Effekte von Calciumcarbonat hinsichtlich der ⁴⁵Ca-Inkorporationsrate.

Verschiedene Rezepturen mit Eischalenpulver in US 3 558 771 wurden insbesondere an Ratten unter topischer Applikation bei offenen Wunden untersucht. Hierbei wurde eine verbesserte Wundheilung im Vergleich mit den Kontroltieren erreicht. In dieser Druckschrift wird kein geeignetes Sterilisierungsverfahren angegeben, das die therapeutische Wirksamkeit der Eischalen nicht beeinflussen würde. Eine orale Applikation von Eischalenpulver ist nicht vorgesehen.

In Chemical Abstracts, Vol. 117, 1992, Ref. 33411x wird Eischalenpulver unter Einwirkung von Milchsäure zu kosmetischen Präparaten verarbeitet. Die auf diese Weise entstehenden Calciumlactatprodukte sind in einen Proteinfilm eingebettet. Diese Lactat-Emulsion wird zu einer kosmetischen Creme verarbeitet. Ähnlich der Präparation 1 (SU 1754 104) enthält diese lediglich bestimmte Fraktionen der Eischale, die topisch appliziert werden.

Es besteht darüber hinaus eine jahrzentelange Erfahrung bei der Herstellung der Spezialitäten - insbesondere bei der Sterilisierung ohne Wirksamkeitsverlust, d. h.: wird die Temperatur von 80°C bei dem Entkeimungsvorgang der Eisschale überschritten, so wird der biologische membranpassagefähige Carrier für die Mineralstoffe zerstört, so daß die Wirkung des thermisch zerstörten Produktes der des Calciumcarbonats hinsichtlich der Inkorparationsrate von ⁴⁵Ca entspricht. Andererseits reicht diese Temperatur allein nicht aus, um den porösen, wärmeisolierenden Rohstoff Eischale/Eischalenpulver vollständig von pathogenen Bakterien, Sporen, Pilzen und Protozoen zu befreien, mit deren Anwesenheit aufgrund der fäkalen Kontamination - insbesondere zusätzlich bei ungünstigen Lagerungsbedingungen - zu rechnen ist.

Erfindungsgemäß wurde gefunden, daß die Verwendung von Putamen ovi gegenüber der Verwendung von reinem Calciumcarbonat bei verschiedenen Krankheitszuständen überraschenderweise Vorteile aufweist. Ein besonderes Problem bei der Verwendung von Putamen ovi als Arzneimittel stellt jedoch die Bereitstellung eines standardisierten sterilen Arzneimittels mit einer definierten Korngröße dar.

In einer ersten Ausführungsform der vorliegenden Erfindung wird die obengenannte Aufgabe gelöst durch ein Verfahren zur Herstellung von Putamen ovi mit einer Korngröße von weniger als 0,1 mm, wobei man
- a): die Eischalen, insbesondere von gallus domesticus bei Raumtemperatur oder erhöhter Temperatur mit Wasser oder einer wässrigen Lösung, enthaltend Desinfizierungsmittel und/oder Tenside unter Rühren wäscht,
- b): die von Schmutzbestandteilen gereinigten Eischalen einem Keimzahlreduzierungsverfahren oder Sterilisierungsverfahren unterwirft,
- c): die Eischalen trocknet und
- d): die Eischalen im Anschluß oder während der Trocknung auf die gewünschte Korngröße zerkleinert.

In der ersten Reinigungsstufe erfolgt die Reinigung durch einfache oder wiederholte Waschung mit gereinigtem Wasser (aqua purificata) möglichst unter Rühren. Ansatzgrößen von 50 bis 100 kg Eischalen erfordern in der Regel etwa 250 l Wasser, wobei der Einsatz von reinigungsaktiven Mitteln, insbesondere Tensiden gegebenenfalls möglich ist. Die in der Reinigung auftretenden Schwebstoffe werden abgezogen und anschließend die gereinigten Eischalen einer Keimzahlreduzierung oder ein Sterilisierungsverfahren unterworfen.

Im Anschluß an die Sterilisierung sowie gegebenenfalls während der Sterilisierung schließt sich die Trocknung der Eischalen bei erhöhter Temperatur an. Hierbei wird insbesondere das in den Poren enthaltene Wasser verdampft. Besonders bevorzugte Trocknungs-Verfahren umfassen die Vakuumtrocknung oder die Gefriertrocknung sowie die Trocknung unter Temperaturerhöhung.

Mit Hilfe der vorliegenden Erfindung ist es möglich, Putamen ovi einer definierten Korngröße herzustellen, bei dem ein Großteil der biologisch aktiven Stoffe, die in der Eischale neben dem Calciumcarbonat enthalten sind, zu erhalten.

Im Stand der Technik sind eine Reihe von Keimzahlreduzierungsverfahren oder Sterilisierungsverfahren bekannt. Dementsprechend besteht eine besondere bevorzugte Ausführungsform der vorliegenden Erfindung darin, das Keimzahlreduzierungsverfahren und das Sterilisierungsverfahren auszuwählen aus Autoklavierung, Heißlufttrocknung, Tyndallisierung, Behandlung mit ionisierender oder nicht ionisierender Strahlung und Gassterilisation.

Besonders bevorzugt im Sinne der vorliegenden Erfindung ist die Heißlufttrocknung bei einer Temperatur oberhalb des Siedepunkts von Wasser, insbesondere bei einer Temperatur von wenigstens 150°C im Verlauf von wenigstens 3 Stunden, bei der Sterilisierung und Trocknung in einem Verfahrensschritt verbunden werden.

Im Anschluß an die Trocknung oder während der Trocknung werden die Eischalen dann auf die gewünschte Korngröße von weniger als 0,1 mm zerkleinert. Besonders bevorzugt im Sinne der vorliegenden Erfindung ist es, die getrockneten Eischalen mit einer Mahlscheibe und anschließender Siebung mit einer Siebgröße von 0,1 x 0,09 mm zu zerkleinern.

In Abhängigkeit vom Einsatz des Mahlaggregates ist es somit möglich, Putamen ovi in einer definierten Korngröße unter weitgehendem Erhalt der aktiven Begleitstoffe bereitzustellen.

Dementsprechend besteht eine weitere Ausführungsform der vorliegenden Erfindung in Putamen ovi, erhältlich nach einem Verfahren wie oben definiert, mit einer Korngrößenverteilung von
> 0,05 mm 35 Gew.-% und
< 0,01 mm 65 Gew.-%.

Das so bereitgestellt Putamen ovi kann unter Verwendung von an sich bekannten Hilfsstoffen und Trägerstoffen zu einem Arzneimittel verarbeitet werden, daß für verschiedenste krankhafte Calciummangel-Zustände eingesetzt werden kann.

Insbesondere wurde gefunden, daß ein so erhältliches Naturstofftherapheutikum neben Calcium in geringen Anteilen weitere lebenswichtige Mineralstoffe wie Eisen, Fluor, Kalium, Kieselsäure, Magnesium enthält. Außerdem enthält es biologisch gebildete organische Wirkstoffe wie Enzyme, Porphyrin, Sterine, Vitamin D3, wie auch die biologisch wichtigen Spurenelement Kupfer, Molybdän, Selen und Zink.

Das Vorliegen von Calcium in biologisch gebundener Form bildet gleichsam eine Schlepperfunktion (Carrier) für die sehr gute Aufnahme aus dem Darm ins Blut. Die gute Resorption der Mineralstoffe, der organischen Wirkstoffe und der Spurenelemente vom Organismus sind Voraussetzung für die deutliche Wirksamkeit von Putamen ovi bei Knochenerkrankungen und damit verbundener Anämie.

Die biologisch wichtigen Spurenelemente fördern die Entwicklung eines gesunden Knochensystems und beeinflußen günstig einen gestörten Knochenstoffwechsel.

Bei Knochenbrüchen wird eine deutliche Verkürzung der Heilung erreicht, die durch eine schnellere Kallus- und Knochenbildung bewirkt wird.

Osteopenie (Abnahme an Knochengewebe) ist keine Erkrankung sondern der altersabhängige Abbau an Knochengewebe, der etwa ab dem 30. Lebensjahr beginnt und im Jahr bis zu 1,5 % betragen kann. Bis zum 70. Lebensjahr tritt so ein Verlust von ca. 1/3 der Knochenmasse ein ohne Gefahr für das Knochenskelett.

Bei unzureichender Calciumzufuhr, Calciumresorptionsstörungen oder Stoffwechselstörungen jedoch entnimmt der Organismus den Knochen Calcium. Diese Entnahme führt zu einer Verminderung der Knochensubstanz. Nicht alle knöchernen Organe werden gleichmäßig bedroht. Zuerst werden die schwammartig aufgebauten Knochen der Wirbelkörper (Wirbelsäule) angegriffen, später erst die Röhrenknochen der Arme und Beine. Besonders bedroht ist deshalb die Wirbelsäule. Wirbelsäulenverformung droht. Die Muskulatur versucht diesen Wirbelsäulenveränderungen entgegenzuwirken. Die zusätzliche Arbeit der Muskulatur führt früher oder später zu Muskelschmerzen. Bei der Osteoporose liegt deshalb ein Muskelschmerz vor.

Das erfindungsgemäß erhältliche Putamen ovi ist daher insbesondere zur Herstellung von Arzneimitteln zur Behandlung von Knochenmarkentwicklungsstörungen, gestörten Knochenmarkfunktionen, Blutbildung, Osteopenie, Osteoporose, Zahnaufbaustörungen, Spasmophilie, Knochenfrakturen, Kallus-Bildung und Kalkmangelerscheinungen während des Wachstums, der Schwangerschaft, Postmenopause und der Stillzeit geeignet.

Die nachfolgenden Ausführungsbeispiele dienen der Erläuterung der Erfindung, ohne diese jedoch darauf zu beschränken.

### Beispiel 1

Herstellung von Putamen ovi.

50 kg Eischalen von gallus domesticus wurden bei 60°C mit 250 l (aqua purificata) 5 mal gewaschen und nach jeder Wäsche die Schwebstoffe abgezogen. Die gereinigten Eischalen wurden zur Keimzahlreduzierung bzw. Sterilisierung in einem Heißluftsterilisator bei 165 °C im Verlauf von 4 Stunden getrocknet.

Die getrockneten Eischalen wurden mit einer Stiftmühle gemahlen und mit einem Sieb einer Maschenweite von 0,1 x 0,09 mm abgetrennt.

Hierbei konnte Putamen ovi mit folgender Korngrößenverteilung erhalten werden:
> 0,05 mm 35 Gew.-% und
< 0,01 mm 65 Gew.-%.

Unter Anwendung an sich bekannter Trägersubstanzen und Citronensäure wurden Dragees hergestellt, die 440 mg Putamen ovi mikronisiert enthalten, entsprechend 160 mg Calciumionen. Die Menge an Citronensäure betrug 1,07 mg.

### Klinische Untersuchen:

Mit den oben hergestellten Dragees wurden klinische Untersuchungen durchgeführt, mit denen die verringerte Knochendichte bei 41 Patientinnen in der Postmenopause untersucht wurde.

Die Dragees wurden 3 mal täglich im Verlauf von 304 Tagen eingenommen. Hierbei wurde eine Zunahme der Knochendichte im Gesamtkollektiv um 9,4 % nach 304 Tagen (durchschnittlich) ermittelt. Die Untersuchungsergebnisse sind Resultate wiederholter osteodensitometrischer Bestimmungen, deren Verlaufskontrollen außerdem durch die Erfassung der biochemischen Marker des Knochenabbaus (Osteoklasten-Aktivität) sowie der Aktivität der Knochenbildung (Osteoblasten-Aktivität) ergänzt wurden.

Die Knochenmineraldichte-Messungen wurden mittels der quantitativen digitalen Radiographie (Hologic QDR-1000 TM bone densitometer) an den Lendenwirbeln (LWK 1-4) durchgeführt, wobei das Ergebnis der Knochenmineraldichteberechnung als Dichte in Gramm Calcium-Hydroxyapatit/cm³ ausgedrückt ist.

Bei diesem osteodensitometrischen Verfahren wurde der Meßwert für die Absorption im Weichteilmantel eliminiert, so daß der Knochenmineralgehalt ohne Weichgewebefehler bestimmt werden konnte. Die Gesamtbilanz auf der Basis von 41 Verlaufsuntersuchungen zeigte eine deutliche Zunahme der Knochenmineraldichte um 9,4 % (von 78,1 % auf 85,5 %).

In einer Gruppe A, die weniger als 200 Tage lang Putamen ovi mit der obengenannten Dosierung eingenommen hatte, stieg die Knochendichte bereits durchschnittlich um 5,5 %. In einer Gruppe B, die weniger als 300 Tage Putamen ovi mit der obengenannten Dosierung eingenommen hatte, stieg der Meßwert auf 7,2 %, während in einer Gruppe C, die länger als 300 Tage Putamen ovi in der obengenannten Dosierung eingenommen hatte, der Meßwert auf die genannten 9,4 % anstieg. Die Ergebnisse zeigten eine deutliche Dosis-Wirkung-Beziehung. Während in einer ersten Gruppe X der Wert unter einer Applikation von 3 x 1 Dragee Putamen ovi um 6,9 % stieg, wurde in einer weiteren Gruppe Y, die 3 x täglich 2 Dragees Putamen ovi eingenommen hatte, eine Knochendichte-Zunahme um 10,9 % gefunden.

Es wurde gefunden, daß bei 12 der untersuchten Patientinnen die Zunahme der Knochendichte unter 5 % lag, bei 18 Patientinnen zwischen 5 und 10 % lag, während bei 11 Patientinnen der Wert über 10 % (+ 15,5 %) lag. Die Responderrate kann somit mit ca. 70 % angenommen werden bei keiner Patientin lag nach Therapieende die Knochendichte unter derjenigen der Erstuntersuchung.

Mit Hilfe der vorliegenden Erfindung ist es offensichtlich möglich, allgemein einer Knochendichteminderung der Wirbelsäule bei Patientinnen in der Postmenopause entgegenzuwirken, auch ohne weitere Substitution mit derzeit im Handel erhältlichen Ostereoporosetherapeutika.

## Patentansprüche

1. Verfahren zur Herstellung von Putamen ovi mit einer Korngröße von weniger als 0,1 mm, wobei man
a) die Eischalen, insbesondere von gallus domesticus bei Raumtemperatur oder erhöhter, Temperatur mit gereinigtem Wasser **(aqua purificata)** unter Rühren wäscht,
b) die von Schutzbestandteilen gereinigten Eischalen einer Autoklavierung unterwirft,
c) die Eischalen trocknet und
d) die Eischalen im Anschluss oder während der Trocknung auf die gewünschte Korngröße zerkleinert.

2. Verfahren nach Anspruch 1, **dadurch gekennzeichnet, dass** man die getrockneten Eischalen mit einer Stiftmühle und anschließender Siebung mit einer Maschenweite von 0,1 mm x 0,09 mm zerkleinert.

3. Putamen ovi, erhältlich nach einem Verfahren wie in Anspruch 1 oder 2 definiert, mit einer Korngrößenverteilung von:
> 0,05 mm 35 Gew.-% und
< 0,01 mm 65 Gew.-%.

4. Verwendung von Putamen ovi gemäß einem oder mehreren der Ansprüche 1 bis 3 **zur Herstellung eines Arzneimittels für eine therapeutische** Behandlung von Knochenmarkentwicklungsstörungen, gestörter Knochenmarkfunktion und Blutbildung, Osteopenie, Zahnaufbaustörungen, Spasmophelie, Knochenfraktur, Kallus-Bildung und Kalkmangelerscheinungen während des Wachstums, der Schwangerschaft, der Postmenopause und der Stillzeit.

## Claims

1. A method for the preparation of putamen ovi having a grain size of less than 0.1 mm wherein
a) egg-shells, especially from *Gallus domesticus,* are washed with purified water (aqua purificata) with stirring at room temperature or elevated temperature;
b) the egg-shells having been cleaned from contaminants are subjected to autoclave treatment;
c) the egg-shells are dried; and
d) the egg-shells are crushed to the desired grain size following or during the drying.

2. The method according to claim 1, **characterized in that** said dried egg-shells are ground with a pin-disk mill followed by screening with a mesh size of 0.1 mm x 0.09 mm.

3. Putamen ovi obtainable by a method as defined in claim 1 or 2 and having a grain size distribution of:
> 0.05 mm 35% by weight; and
< 0.01 mm 65% by weight.

4. Use of putamen ovi according to one or more of claims 1 to 3 for the preparation of a medicament for therapeutic treatment of bone marrow development disorders, bone marrow dysfunctions and dyshematopoiesis, osteopenia, dental build-up disorders, spasmophilia, bone fractions, callus formation and calcium deficiency symptoms during growth, pregnancy, postmenopause and nursing period.

## Revendications

1. Procédé de production de Putamen ovi, ayant une granulométrie d'au moins 0,1 mm, dans lequel
a) on lave sous agitation les coquilles d'oeuf, en particulier de Gallus domesticus, à la température ambiante ou à une température plus élevée, avec de l'eau purifiée (aqua purificata),
b) on soumet à un autoclavage les coquilles d'oeuf débarrassées des constituants protecteurs,
c) on sèche les coquilles d'oeuf et,
d) on broie les coquilles d'oeuf, après ou pendant le séchage, jusqu'à la granulométrie voulue.

2. Procédé selon la revendication 1, **caractérisé en ce qu'**on broie les coquilles d'oeuf séchées à l'aide d'un moulin désintégrateur, puis on les tamise à l'aide d'un tamis ayant une ouverture de maille de 0,1 mm x 0,09 mm.

3. Putamen ovi, pouvant être obtenu par un procédé selon la revendication 1 ou 2, ayant la distribution granulométrique suivante : > 0,05 mm 35 % en poids et < 0,01 mm 65 % en poids.

4. Utilisation de Putamen ovi selon l'une ou plusieurs des revendications 1 à 3 pour préparer un médicament destiné à un traitement thérapeutique des troubles du développement de la moelle osseuse, d'un trouble de la fonction de la moelle osseuse et de l'hématopoïèse, de l'ostéopénie, des troubles de la formation des dents, de la spasmophilie, des fractures osseuses, de la formation de cals et de phénomènes d'hypocalcémie pendant la croissance, la grossesse, la post-ménopause et la lactation.
